# EUROPEAN PATENT APPLICATION

(11) **EP 1 767 946 A1**
(43) Date of publication of application: **28.03.2007**
(21) Application number: 05291967.7
(22) Date of filing: 22.09.2005
(51) Int. Cl.: G01N 33/68

(54) **Process of screening a molecule as a candidate drug for the treatment of bipolar disorder**

(71) Applicant: INSTITUT PASTEUR, 75015 Paris (FR); Centre National de la Recherche Scientifique (CNRS), 75016 Paris (FR)
(72) Inventor: Mechold, Undine, 92350 Le Plessis Robinson (FR); Ogryzko, Vasily, 92350 Le Plessis Robinson (FR); Danchin, Antoine, 75013 Paris (FR)
(74) Representative: Warcoin, Jacques

(57) **Abstract**

The invention relates to a complex formed between the 3'-phosphoadenosine 5'-phosphate and polypeptide in human cells, to the use of pAp or of a molecule which mimics pAp for modulating the activity of poly(ADP-ribose)polymerase and small fragment nuclease in human cells, and to process of screening a molecule as a candidate drug for the treatment of diseases where modulation of at least one of the metabolic activities modulated by pAp is needed such as bipolar disorder or related diseases.

## Description

The present invention is directed to complexes formed with 3'-phosphoadenosine 5'-phosphate (pAp) metabolite, to the use of pAp or of a molecule which mimics pAp and to process of screening a molecule as a candidate drug for the treatment of diseases, where modulation of at least one of the metabolic activities modulated by pAp is needed, such as bipolar disorder or related diseases.

Mental disorders encompass a wide range of central nervous system disorders. Mental disorders include, e.g., mood disorders, psychotic disorders, anxiety disorders, childhood disorders, eating disorders and personality disorders, all these terms being defined according to the DSM-IV classification (Diagnosis and Statistical Manual of Mental Disorders, Fourth Edition, American Psychiatric Association, Washington D.C., 1994). Mood Disorders encompass bipolar 1 disorder (mania with or without major depression), bipolar 11 disorder (hypomania with major depression), cyclothymic disorder (numerous brief episodes of hypomania and minor depression), dysthymic disorder (prolonged minor depression without mania/hypomania) and major depressive disorder (major depression without mania).

Bipolar disorders are relatively common disorders, occurring in about 1.3% of the population, and have been reported to constitute about half of the mood disorders seen in psychiatric clinics with severe and potentially disabling effects. Bipolar disorders have been found to vary with gender depending of the type of disorder; for example, bipolar disorder 1 is found equally among men and women, while bipolar disorder 11 is reportedly more common in women. The age of onset of bipolar disorders is typically in the teenage years and diagnosis is typically made in the patient's early twenties. Bipolar disorders also occur among the elderly, generally as a result of a neurological disorder of other medical conditions. In addition to the severe effects on patients' social development, suicide completion rates among bipolar patients are reported to be about 15%.

Bipolar disorders are characterized by phases of excitement and often depression; the excitement phases, referred to as mania or hypomania, and depressive phases can alternate or occur in various admixture, and can occur to different degrees of severity and over varying duration. Since bipolar disorders can exist in different forms and display different symptoms, the classification of bipolar disorder has been the subject of extensive studies resulting in the definition of bipolar disorder subtype and widening of the overall concept to include patients previously thought to be suffering from different disorders.

Bipolar disorders often share certain clinical signs, symptoms, treatments and neurobiological features with psychotic illnesses in general and therefore present a challenge to the psychiatrist to make an accurate diagnosis. Furthermore, because the course of bipolar disorders and various moods and psychotic disorders can differ greatly, it is critical to characterize the illness as early as possible in order to offer means to manage the illness over a long term.

The mania association with the disease impairs performance and causes psychosis, and often results in hospitalization. The disease places a heavy burden on the patient's family and relatives, both in terms of the direct and indirect costs involved and the social stigma associated with the illness, sometimes over generations. Such stigma often leads to isolation and neglect. Furthermore, the earlier the onset, the more severe are the effects on interrupted education and social development.

Depressive episodes may be treated like depression. However, most antidepressants can cause swings from depression to hypomania or mania and sometimes cause rapid cycling between them. Therefore, these drugs are used for only short periods, and their effect on mood is closely monitored. At the first sign of a swing to hypomania or mania, the antidepressant is stopped. Most people with manic-depressive disorder are given drugs with a mood-stabilizing effect such as lithium, carbamazepine and divalproex.

Lithium has no effect on normal mood but reduces the tendency toward mood swings in about 70% of the people with manic-depressive illness. A doctor monitors the level of lithium in the blood with blood tests. Possible adverse effects of lithium include tremor, muscle twitching, nausea, vomiting, diarrhea, thirst, excessive urination, and weight gain.

Lithium can make acne or psoriasis worse, can cause the blood levels of thyroid hormone to fall, and rarely can cause excessive urination. A very high level of lithium in the blood can cause a persistent headache, mental confusion, drowsiness, seizures, and abnormal heart rhythms. Adverse effects are more likely to occur in the elderly. Women who are trying to become pregnant must stop taking lithium, because lithium may cause heart defects in a developing fetus.

Newer drug treatments have evolved over the past several years. These include the carbamazepine and divalproex. However, carbamazepine can seriously reduce the number of red and white blood cells, and divalproex can cause liver damage (primarily in children).

Since lithium is known to be toxic and that it is difficult to follow the treatment, and since other treatments have side effects and act only against the symptoms of the disease as well, there is a strong need for new molecules without associated side effects that are specifically directed against the targets which are involved in the mechanisms of treatment, including prevention, of bipolar disorder. Therefore, the inventors have identified new complexes of molecules involved in the lithium metabolic effect providing targets involved in treatment, including prevention, of bipolar disorder. Those new targets allow screening for new drugs efficient in treatment, including prevention, of bipolar disorder, or treatment, including prevention, of side effects linked to lithium administration.

Therefore, a first aspect of the present invention is directed to a complex formed between the 3'-phosphoadenosine 5'-phosphate (pAp) and a polypeptide in human cells.

In a preferred embodiment, the polypeptide is the Poly (ADP-ribose) Polymerase (PARP).

In another preferred embodiment, the polypeptide is the Small fragment nuclease (Sfn).

In still another preferred embodiment, the complex is a purified complex.

A second aspect of the present invention is directed to the use of a molecule which modulates the formation of the pAp-PARP complex according to or pAp-S% complex according to the first aspect of the invention, for the manufacture of a medicament for preventing or treating bipolar disorders and/or side effects linked to lithium administration.

A third aspect of the present invention is directed to a process for screening a molecule which modulates or mimics at least one effect of lithium in human, said process comprising:
a) incubating a polypeptide which complexes with pAp in human cells with said molecule,
b) measuring the activity of said polypeptide,
wherein a modulating or mimic of the activity of said polypeptide indicates that said molecule modulates or mimics at least one effect of lithium in human.

In a preferred embodiment, the process for screening a molecule mimics at least one effect of lithium in human, and said process comprises:
a) incubating a polypeptide which complexes with pAp in human cells with said molecule,
b) measuring the activity of said polypeptide,
wherein a modulating of the activity of said polypeptide which is comparable with the modulating of the activity of said polypeptide in the presence of pAp in the cell indicates that said molecule mimics at least one effect of lithium in human.

In another preferred embodiment, the process for screening a molecule modulates at least one effect of lithium in human, and said process comprises:
a) incubating a polypeptide which complexes with pAp in human cells with said molecule in the presence of pAp in the cell,
b) measuring the activity of said polypeptide,
wherein a modulating of the activity of said polypeptide compared with the activity of said polypeptide in the presence of pAp in a cell indicates that said molecule modulates at least one effect of lithium in human.

In still other preferred embodiments :
- the presence of Pap in the cell derived from the presence of lithium,
- said polypeptide is PARP,
- said effect of lithium in human is degradation of RNA-oligos and/or DNA-oligos,
- said polypeptide is Sfn,
- said effect of lithium in human is modulation of DNA repair.
- the molecule is a candidate drug for the treatment of bipolar disorder.

A fourth aspect of the present intention is directed to the use of a molecule screened in the process according to the third aspect of the invention as a medicament.

In a preferred embodiment, the molecule is used for the manufacture of a medicament for treatment of bipolar disorder.

A fifth aspect of the present invention relates to the use of pAp or of a molecule which mimics pAp for modulating DNA repair and/or for degrading RNA-oligos and/or DNA-oligos.

In a preferred embodiment, the use is for the manufacture of a medicament for preventing or treating diseases where a modulation of PARP activity is needed, such as cancer, cell necrosis, inflammation, neurodegenerative diseases or tissue injury.

In other preferred embodiments, in the use:
- PARP activity is inhibited,
- Sfn activity is modulated, and
- the manufacture of a medicament is for preventing or treating bipolar disorders and/or side effects linked to lithium administration

Further, a sixth aspect of the present invention pertains to a downstream target polypeptide of pAp in human cells for use as a medicament.

In a seventh aspect, the present invention relates to the use of the downstream target polypeptide of pAp according to the sixth aspect of the invention for the manufacture of a medicament for treating bipolar disorder.

In a preferred embodiment, the downstream target polypeptide of pAp is selected from Sfn and PARP.

In the present invention, the term "complex" means that a polypeptide binds to pAp with high specificity and therefore forms a complex where pAp can modulate, including inhibit or activate, the metabolic activity of the polypeptide.

The term "purified" requires that the material be removed from its original environment (e.g., the natural environment). For example, a naturally-occurring protein present in a living animal is not isolated, but the same protein, separated from some or all of the coexisting materials in the natural system, is isolated. It means that the indicated complex (formed from pAp and a polypeptide according to the first aspect of the present invention) is present in the substantial absence of other biological macromolecules of the same type. The "purified" as used herein preferably means at least 75% by weignt, more preferably at least 85% by weight, more preferably still at least 95% by weight, and most preferably at least 98% by weight, of biological macromolecules of the same type present (but water, buffers, and other small molecules, especially molecules having a molecular weight of less than 1000, can be present).

The term "polypeptide" refers to a polymer of amino acids without regard to the length of the polymer. Thus, peptides, oligopeptides and proteins are included within the definition of polypeptide. This term also does not exclude post-expression modifications of polypeptides, for example, polypeptides which include the covalent attachment of glycosyl groups, acetyl groups, methyl groups, phosphate groups and the like are expressly encompassed by the term polypeptide. Also included within the definition are polypeptides which contain one or more analogs of amino acid (including, for example, non-naturally occurring amino acids, amino acids which only occur naturally in an unrealated biological system, modified amino acids from mammalian systems etc.), polypeptides with substituted linkages, as well as other modifications known in the art, both naturally occurring and non-naturally occurring.

The term "treatment" as used herein means to ameliorate, alleviate symptoms, eliminate the causation of the symptoms either on a temporary or permanent basis, or to prevent or slow the appearance of symptoms of the name disorder or condition.

The term "bipolar disorder" as used herein refers to a condition characterized as a Bipolar Disorder in the DSM-IV. Bipolar disorder may be bipolar 1 and bipolar 11 as described in the DSM-IV. The term further includes cyclothimic disorder. Cyclothimic disorder refers to an alternation of depressive symptoms and hypomanic symptoms. The skill person in the art will recognize that there are alternative nomenclatures, posologies, and classification systems for pathologic psychological conditions and that these systems evolve with medical scientific progress.

In the present invention, the term "downstream target polypeptide of pAp" means specifically polypeptide which binds downstream from pAp, and which has pAp as a substrate or that is positively or negatively regulated by pAp.

As used herein, "an effect of lithium" is the modulation of the metabolic activity of a downstream target of pAp. Preferably, an effect of lithium is the modulation of PARP or of Sfn activity.

As used herein, "a molecule which modulates at least one effect of the lithium" is a molecule which modulates, either positively or negatively, the metabolic activity of a downstream target of pAp, or the modulation of the metabolic activity of a downstream target of pAp. The modulation of an effect of the lithium can be, for example, an activation of a downstream target of pAp, an inhibition of a downstream target of pAp, and increase of the activation of a downstream target of pAp, a decrease of the activation of a downstream target of pAp, a decrease of the inhibition of a downstream target of pAp or an increase of the inhibition of a downstream target of pAp. Said modulation of one effect of the lithium is defined with comparison with either the metabolic activity of a downstream target of pAp in a cell or the modulation of the metabolic activity of a downstream target of pAp in a cell in the presence of lithium.

The beneficiary effect of lithium on patients with bipolar disorder was described for the first time more than 50 years ago (Cade, 1949). Efforts to identify targets of lithium concentrated to a large extent on its action on glycogen synthase kinase (GSK)-3 (Klein & Melton, 1996; Stambolic et al, 1996) and inositol monophosphatase (Berridge et al, 1989; Hallcher & Sherman, 1980). Those and other hypotheses are reviewed (Quiroz et al, 2004; Shaldubina et al, 2001).

While the basic building blocks of the cell are usually considered when studying intermediary metabolism, some are overlooked. 3'-phosphoadenosine 5'-phosphate (pAp) in the metabolism of sulfur is a case in point. It is a ubiquitous 3'-phosphorylated nucleotide derived from sulfur assimilation. pAp is generated from phosphoadenosine 5'-phosphosulfate (PAPS), an intermediate step in sulfate assimilation, by two different mechanisms, as a by-product of either: I) sulfonation reactions, or II) reduction to sulfite. Sulfonation, catalyzed by numerous sulfotransferases is the transfer of sulfate to a large number of acceptor molecules that play important roles in mammalian cells; in addition to its effects on structure and function of macromolecules, sulfonation is also involved in deactivation and bioactivation of hormones, neurotransmitters, xenobiotics, and elimination of end products of catabolism (Falany, 1991; Schneider et al, 1998; Weinshilboum & Aksoy, 1994; Weinshilboum, 1986). Reduction of PAPS to sulfite is part of the pathway that leads to the biosynthesis of cysteine and methionine in microorganisms, a pathway that is absent in humans. It is catalyzed by PAPS reductase with liberation of pAp in the cell. Furthermore, and independent from sulfur metabolism, pAp is also generated from coenzyme A during the transfer of the 4-phosphopantetheine group to acyl carrier protein (ACP) in fatty acid synthesis or to secondary metabolites such as peptide antibiotics, surfactin or polyketides (Reuter et al, 1999).

pAp needs to be recycled to AMP, which is accomplished by a 3'-nucleotidase. The question arises whether this nucleotide is solely a metabolic intermediate or whether it has regulatory function as well. pAp is known to act as competitive inhibitor of enzymes that use PAPS, mainly PAPS reductase and sulfotransferases (Klaassen & Boles, 1997); however, other targets could also exist. The importance of the search for pAp targets is attested by the fact that the enzymes degrading pAp belong to a structurally conserved protein family that is uniquely inhibited by lithium (York et al, 1995).

In the present invention, the inventors have focused on lithium's action on members of a structurally conserved family of metal dependent phosphomonoesterases that include inositol monophosphatase (York et al, 1995) and more particular on pAp phosphatase.

The invention shows that at least one indirect target of lithium treatment is remarkably evolutionary conserved, suggesting that the role of pAp is probably more important than previously suspected.

The inventors have investigated regulatory downstream target polypeptides binding to pAp, starting from the model bacterium *Escherichia coli.*

PAp-accumulation due to the inhibition of pAp-phosphatase could explain some of the effect of lithium treatment. The inventors have therefore started a systematic search for regulatory targets of this 3'-phosphorylated nucleotide. The approach of the inventors was to identify polypeptides binding to pAp by affinity chromatography. They expected to find polypeptides that have pAp as a substrate or polypeptides that are regulated by pAp, in order to get regulatory downstream target polypeptides of pAp, to form complex with pAp. With CysQ, the inventors have found a protein that uses pAp as substrate and cleaves the 3' phosphate, leaving AMP as a metabolite directly recycled in metabolism by adenylate kinase (Spiegelberg *et al.,* 1999). The inventors demonstrate that CysQ, like other members of a structurally conserved protein family, is highly sensitive to lithium and calcium in vitro. Furthermore, CysQ is not inhibited by physiological concentration of sodium (200mM) like mammalian pAp-phosphatases rat BPNT1 (Lopez-Coronado et al, 1999) and human BPNT1 (Yenush et al, 2000) and two out of three homologous proteins in Arapidopsis (At-Sal1 and At-Sal2) (Gil-Mascarell et al, 1999) and unlike the yeast proteins Met22/Hal2P (Murguia et al, 1996) and Tol1 (Miyamoto et al, 2000) and At-AHL (Gil-Mascarell et al, 1999).

The inventors have found that CysQ is a main target of lithium-toxicity in *E. coli* as overexpression of this protein can almost completely rescue the growth defect observed when bacterial cells are treated with LiCl.

The inventors' search for pAp-binding proteins in *E. coli* has also revealed an exonuclease, Orn, the activity of which is directed from 3' to 5'. This oligoribonuclease binds tightly to pAp and is inhibited by micromolar amounts of pAp in vitro. Remarkably, when using HeLa cell extract, the inventors have found the human homologue of Orn, Sfn to bind to pAp-agarose. Both proteins were shown to degrade distinctively small oligoribonucleotides shorter than 5 nucleotides in vitro. Orn is the only *E. coli* exoribonuclease that is able to degrade small oligoribonucleotides (Yu & Deutscher, 1995), and short oligoribonucleotides (2-5 nucleotides) were shown to accumulate under circumstances of a conditional knockout (Ghosh & Deutscher, 1999). It is encoded by an essential gene, which is unusual for *E.coli* exoribonucleases. Ghosh and Deutscher consider three different explanations why the absence of Orn activity leads to cessation of growth: 1) depletion of the cells' mononucleotide pool, 2) inhibition of certain enzymes of essential metabolic processes by accumulated oligoribonucleotides, or 3) a yet unknown function of Orn that is essential. In this respect, it is interesting that Orn activity was shown to display some degree of sequence specificity (Datta & Niyogi, 1975), and therefore, its function could be targeted to a more specific set of short oligoribonucleotides. The role of small RNAs, microRNAs and siRNAs in the regulation of gene expression is increasingly recognized and has seen enormous advances in the last decade. It is conceivable that short oligos could interfere with the metabolism of those small RNAs by means of hybridyzation and thereby contribute to changes in gene expression. It is introduced the term nanoRNAs in the present invention to distinguish them from the longer microRNAs.

In the present invention, "RNA-oligos" means short RNA-oligos such as nano-RNA.

The human Orn homologue (Sfn) (Nguyen *et al.,* 2000) was similarly identified as pAp-target with extracts from HeLa cells pointing to conservation of at least one indirect lithium target from prokaryotes to mammals. Sfn and Orn share a high degree of similarity (50% identity) and have conserved function. As Orn, Sfn specifically degrades short oligoribonucleotides of ≤ 5 nucleotides in length.

Sfn was shown to degrade small DNA-oligos in addition to oligoribonucleotides (Nguyen et al, 2000). These short DNA-oligos could emerge from DNA repair and recombination and indeed it was shown that Sfn plays a role in resistance to UV-C (Ito et al, 2004). Both Orn and Sfn belong to the 3'-5' exonuclease superfamily, which includes proofreading domains of all known DNA-dependent DNA polymerases (Koonin, 1997), therefore Orn could degrade short DNA oligos as well, a possibility that was never tested when this enzyme was characterized as a ribonuclease, only double stranded T7 DNA was used as a DNA substrate (Niyogi & Datta, 1975). The inventors therefore tested the bacterial Orn for activity on oligo-DNA (5 nucleotides) and found that like Sfn, Orn is able to degrade DNA.

Another polypeptide the inventors have found to bind to pAp is nucleoside diphosphate kinase (Ndk). The inhibition of Ndk by 3'-phosphorylated nucleotides, in particular pAp and PAPS was already reported (Schneider et al, 1998). Thus the inventors' finding of Ndk in the pAp-binding fraction validates the method and supports the physiological significance of this inhibition. In addition to maintenance of (d)NTP levels, Ndks were suggested to have other important metabolic functions in processes such as signal transduction (Bominaar et al, 1993) and developmental control (Biggs et al, 1990).

In humans there exist eight different and widely expressed Ndks, called NM23-H1 to NM23-H8 some of which play a critical role in development (Lacombe et al, 2000; Lombardi et al, 2000; Postel et al, 2000b; Venturelli et al, 1995). Nm23-H1 and H2, the human Ndks identified in the screen for pAp-binding proteins of the present invention, are the most abundant and closely related (88% identity) isoforms. The two Ndk homologues were shown to act as suppressor of tumor metastasis but also to function in tumorogenesis, (Hartsough & Steeg, 2000; Postel, 1998). NM23-H2 binds specifically to the promoter of the c-myc oncogene and activates its transcription (Postel et al, 1993). This activation involves structural rearrangements through cleavage of a nuclease hypersensitive region (NHE) (Postel, 1999). Of interest for pAp-inhibitory effect is the fact that the residue required for this cleavage reaction, Lys 12, lies in the catalytic pocket involved in the nucleoside diphosphate (NDP) kinase phosphorylation reaction (Postel et al, 2000a). pAp binds to Ndk at the same site as the physiological substrates (d)NTP or (d)NDP without disturbing the confirmation of the active site in an inverted position where the 3' phosphate occupies the space the γ-phosphate of ATP would reside in (Schneider et al, 1998) close to the catalytic histidine. ATP but not ADP was shown to inhibit the cleavage reaction with 50% inhibition at 0.5mM (Postel et al, 2002). Considering the fact that it is specifically the γ-phosphate and the adenine base that inhibits the cleavage and not ATP hydrolysis or phosphorylation of the catalytic histidine, the inventors predict that pAp will inhibit not only NDP kinase activity but also DNA cleavage required for transcription activation of c-myc as well as repair (Postel & Abramczyk, 2003). This is the more likely as pAp makes polar interactions with Lys12 (Schneider et al, 1998) required for cleavage. A most interesting feature of this remarkably conserved role is that it links sulfur metabolism to completely unexpected targets.

A third polypeptide the inventors have found is PARP. PARP was implicated in the pathogenesis of several important diseases, such as stroke, myocardial infarction, diabetes, shock, neurodegenerative disorders, allergies and inflammatory processes. PARP-inhibitors are therefore of pharmacological interest (Jagtap *et al.,* 2005). Inhibitors of PARP can be used for the prevention or treatment of cancer, cell necrosis like in stroke or myocardial infarction, inflammation and tissue injury, for example. PARP is activated by DNA damage and uses NAD+ to modify a variety of proteins post-translationally adding a poly ADP-ribose tail that can be up to 200 residues and branched. This modification changes the activity of the acceptor protein and leads to changes in DNA replication, transcription and repair. Under conditions of mild DNA damage, PARP activity helps to recruit the repair machinery leading to successful repair and increased cell survival. However, overactivation of PARP leads to depletion of ATP and NAD and subsequently to necrotic cell death. As lithium was shown to have neuroprotective effects, as were inhibitors of PARP (Cimarosti *et al.* (2201), Skaper et al. (2003, October and May)), the effects of pAp on PARP activity might explain the neuroprotective effects of lithium.

The method of identifying pAp-binding polypeptides is not exhaustive and the inventors expect it to be restrictive to proteins with higher expression levels. The knowledge of pAp-targets improves the molecular understanding of the multivarious therapeutical and toxical effects of lithium and leads to more directed pharmacological approaches. The present invention suggests the existence of a network of polypeptides that are controlled by pAp and/or pAp levels, a network that exists independent and in addition to the better explored glycogen synthase-3 (GSK-3), and protein kinase C networks (Lenox & Wang, 2003), and a network that contributes significantly in the mechanisms of lithium action.

The figures of the present invention show the results of the different examples hereafter described.
**Figure 1**: pAp binding proteins from *E. coli.* Shown is colloidal coomassie stained SDS-PAA gel separating the pAp binding fraction of *E.coli* extract. M: marker, lane 1: control, lane 2: pAp-binding fraction.
**Figure 2:** Inhibition of recombinant CysQ by lithium (1mM) and calcium (2mM). Black circles: no inhibitor; gray circles: LiCl; white squares: CaCl₂.
**Figure 3:** Lithium induced growth inhibition in *E. coli* MG1655 containing pBAD18 (vector control) or pUM404 (arabinose-inducible cysQ). Strains were grown on MOPS-minimal plates containing all amino acids but cysteine and methionine, 0.4% glycerol, and 0.0002% arabinose.

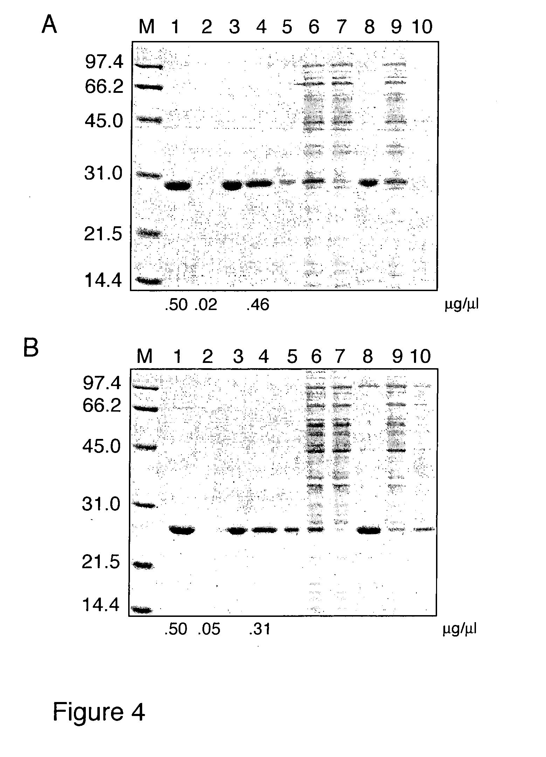

**Figure 4**: Preferential binding of Orn to pAp as compared to AMP. M: marker. A: recombinant CysQ and B: recombinant Orn; lanes 1: purified enzyme-input; lanes 2: flow through pAp agarose; lanes 3: eluate pAp-agarose; lanes 4: flow through AMP-agarose; lane 5: eluate AMP-agarose; lanes 6: bacterial cell extracts of strains overexpressing CysQ or Orn, respectively; lanes 7: flow through of bacterial extracts on pAp-agarose; lanes 8: eluate of bacterial extracts on pAp-agarose; lanes 9: flow through of bacterial extracts on AMP-agarose; lanes 10: eluate of bacterial extracts on AMP-agarose. Amounts loaded are 1 µl for lanes 6, 7 and 9; 2.5 µl for lanes 1-5, 8A, and 10A; 10 µl for lanes 8B and 10B. Numbers below the gels correspond to protein concentrations measured in the according fraction.
**Figure 5**: pAp is a stronger inhibitor of Orn activity as compared to AMP. A: SDS-PAA gel electrophoresis for the separation of reaction products. B: quantification of data shown in A. closed circle: 5mer, open circle: 4mer, closed triangle: 3mer, open triangle: 2mer, square: 1mer.
**Figure 6**: pAp binding proteins from humans. Shown is colloidal coomassie stained SDS-PAA gel separating the pAp binding fraction of HeLa cytoplasmic extract. M: marker, lane 1: control, lane 2: pAp-binding fraction.
**Figure 7**: human proteins binding to pAp-agarose.

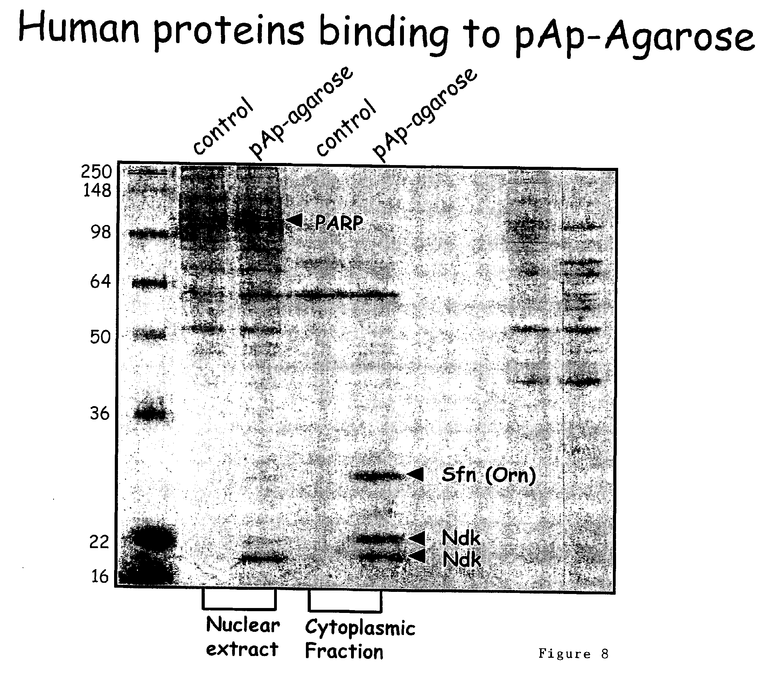

**Figure 8**: changed pattern of ADP-rybosylation in HEK293 cells treated with LiC1.

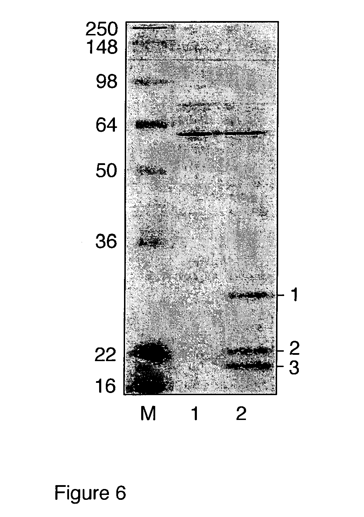

The sequence listing herein enclosed shows the peptides found in figure 6 presented in example 2.

The following examples illustrate the present invention but are not limiting the scope of the present invention.

### Material and Methods used in the following examples :

### 1 - Strains, plasmids, growth conditions

*E. coli* strains were grown at 30°C in MOPS minimal medium (Neidhardt et al, 1974) containing 40 µg/ml of amino acids as indicated, K-phosphate at 2mM, vitamin B1 at 0.0005%, glycerol at 0.4%, glucose or arabinose as indicated. Ampicillin (100 µg/ml) was added for plasmid maintenance. CC118 is disclosed in Manoil & Beckwith, 1985. All experiments were performed in accordance with the European regulation requirements concerning the use of Genetically Modified Organisms (level 1 containment, agreement n°2735).

CysQ was cloned under control of the arabinose-inducible promoter Pₐᵣₐ by replacing the NheI/XhoI fragment coding for RelSeq 1-224 from pUM131 by a PCR fragment containing the open reading frame for cysQ, amplified from MG1655 chromosomal DNA using primer UM133 and UM131. Orn was cloned likewise using primer UM143 and UM139. Chromosomal DNA for use as PCR template was prepared using FTA cards (Whatman).

The microorganism *E. coli* MG1655 containing cysQ gene referenced pUM404 has been deposited on September 14, 2005 in the Collection Nationale de Cultures de Microorganismes under number I-3497.

The microorganism *E. coli* MG1655 containing Orn gene referenced pUM408 has been deposited on September 14, 2005 in the Collection Nationale de Cultures de Microorganismes under number I-3498.

### 2 - Purification of His-tagged CysQ and Orn

CysQ and Orn were purified from 100ml cultures of MG1655 carrying the appropriate plasmid, grown at 30 °C to an A₆₀₀ between 0.7 and 0.9, and then induced for 3 hours with 0.2% arabinose. Cells were harvested and washed once with 50mM Na-phosphate, 300mM NaCl before freezing. Frozen pellets were resuspended in 1.5ml binding buffer (50mM Na-phosphate, 300mM NaCl, 10mM imidazole, 0.4mM PMSF) and lysed by incubation on ice for 30 minutes with 130 µg/ml lysozyme followed by sonication. After removal of cell debris by centrifugation at 14,000 rpm for 30 minutes, proteins were batch purified on 500µl Ni-agarose (Quiagen) according to the instructions of the supplier. Wash buffer contained 20 mM imidazole. Elution was done using four aliquots of 500 µl of elution buffer (same as binding buffer, but containing 250 mM imidazole). The most pure fraction was identified by SDS-PAA gel electrophoresis, dialyzed against 50mM HEPES pH7.5, 100mM NaCl and kept on ice for storage.

### 3 - pAp agarose binding

Cyanogen bromide activated agarose beads (Sigma-Aldrich) were blocked with 0.2 M glycine according to the instructions of the supplier and stored at 4 °C in 1M NaCl containing 0.02% sodium azide. AMP- and pAp-agarose (Sigma-Aldrich) was stored likewise after swelling and washing in H₂O as indicated in the protocol of the manufacturer. Before use, agarose beads were washed 3 times with at least 10 volumes of pAp-agarose buffer (as in (Spiegelberg et al, 1999): 50 mM HEPES pH7.5, 10mM CaCl₂, 50 mM KCl). 200ml cultures of CC118 were grown at 30°C to an A₆₀₀ between 0.7 and 0.8 in MOPS minimal medium containing all amino acids but cysteine and methionine and 2% glucose, harvested and washed once in 50mM Na-phosphate, 300mM NaCl before freezing. Frozen pellets were resuspended in 2 ml pAp-agarose buffer containing 0.4 mM PMSF and lysed as described above. Debris-free cellular extract was added to 200 µl blocked agarose beads and rotated for 2h at 4 °C to clear the extract from proteins binding non-specifically to agarose. Supernatant was divided equally and added to 50 µl of washed pAp-agarose (pAp-binding fraction) or blocked agarose beads (control). After rotation at 4 °C for 1h30 minutes, the beads were washed extensively (10 times 800µl) with pAp-agarose wash buffer (as pAp-agarose buffer but 0.5M NaCl), and washed once with pAp-agarose buffer to remove excessive salt. Elution was done by adding 75 µl of hot SDS-sample buffer, and incubation at 65 degrees for 15 minutes. Beads were removed by centrifugation, and 20 µl of each sample was analyzed by Gel-electrophoresis on 12% SDS-PAA gels. Bands corresponding to proteins binding specifically to pAp were localized by staining with Bio-Safe colloidal coomassie (Bio-Rad), cut out and digested in-gel by trypsin after reduction and alkylation according to standard protocols and analysed by nano-HPLC (LC Packing) directly coupled to an ion trap mass spectrometer (ThermoFinnigan LCQ Deca XP) equipped with a nanoelectrospray source (LC-MS/MS). Database searches and evaluation of the results was done using the Bioworks 3.1 software. Overall scores were obtained using a multiple threshold filter for individual peptides: 1.5 for one charge, 2 for doubly charged, 2.5 for triply charged ions.

For pAp-binding experiments with human cells, HeLa cells were grown in spinner flasks in DMEM including 7% of FBS. 100 million cells were used to prepare cytoplasmic extract according to Dignam (Dignam et al, 1983).

### 4 - Orn activity assay

Custome made RNA-oligo 5mers (5'Cy5-CCCCC3'), obtained from Proligo, were used as substrate for *in vitro* activity assays. 30 µl reactions containing 50mM HEPES pH7.5, 5mM MnCl₂, 3µM substrate RNA and 0.1µg oligoribonuclease were incubated at 37°C, and reactions were initiated by adding substrate. Inhibitors were added as indicated in the text. At intervals, 5 µl reaction aliquots were taken and stopped by adding an equal volume of sample buffer (95% formamide, 20mM EDTA, 0.1 % bromphenol blue) and frozen at - 20 °C. For analysis of the reaction products, samples were heated at 80°C for 3 minutes and 2.5 µl was applied to PAGE on a 22% SDS-PAA gel containing 2xTBE and run in 2xTBE. Fluorescent RNA oligos were visualized using a Molecular Dynamics STORM 860 in 650-nm long-pass filter mode.

### 5 - CysQ activity assay

CysQ activity was tested in 30 µl reactions containing 50mM HEPES pH7.5, 50mM KC1, 200mM NaCl, 2mM MgCl₂, 5.5mM pAp at 37°C, reactions were started by the addition of 0.5 µg CysQ. Inhibitors were added as indicated. At intervals, 4.5 µl samples were mixed with 0.5 µl 100mM EDTA, and resolved by polyethyleneimine (PEI) thin-layer chromatography with 0.8M LiC1 as solvent. Authentic AMP and pAp were used as migration standards. Accumulation of reaction products was estimated after visualization by UV.

### 6 - pAp/AMP-binding assay

30 µl samples containing 15µg of Orn-His or CysQ-His in pAp-agarose buffer containing 100mM NaCl were incubated with 5µl of prewashed pAp- or AMP-agarose by rotating for 1 hour at 4°C in durapore spin columns (Millipore). Beads were removed by centrifugation, and 5µl of the unbound fraction, designated flow through was measured for protein content using Bio-Rad protein assay. The fraction bound to the beads was eluted by the addition of 40µl of hot SDS-sample buffer and incubation for 15 minutes at 65°C. Similarily, 60 µl of cell-free extracts of MG1655 overexpressing CysQ (pUM404) or Orn (pUM408) obtained as described under protein purification and dialyzed against pAp-agarose buffer containing 100mM NaCl were used for comparable binding experiments.

7 - Table I presents the strains, plasmids and primers that are used in the present invention.

**Table I: Strains, plasmids, and primers.**

| **strains** | | |
|---|---|---|
| MG1655 | E. coli K-12, wildtype | (Bachmann, 1996) |
| CC118 | [D(ara-leu)7697 araD139 DlacX74 galE galK DphoA20 thi-1 rpsE rpoB argE(Am) recA1] | (Manoil & Beckwith, 1985) |

| **plasmids** | | |
|---|---|---|
| pBAD 18 | vector, BAD po; pBR replicon; Ap^{R} | (Guzman et al, 1995) |
| pUM404 | as pBAD18, CysQ; C-terminal His tag | CNCM I-3497 |
| pUM408 | as pBAD18, Orn; C-terminal His tag | CNCM I-3498 |

| **primers** | | |
|---|---|---|
| UM131 | 5'GGGGCTCGAGgtaaatagacactctgaaccccg3' | |
| UM133 | 5'GGGGCTAGCAAGAAGGAGATATACCatgttagatca agtatgccagcttgcac3' | |
| UM139 | 5' GAGAGGTC GACcagcttgataaaatgctcgcggtagt3' | |
| UM143 | 5'GGGGCTAGCAGGAGGAATTCACCatgagtgccaatg aaaacaacctgatttggat3' | |

### EXAMPLE 1: Identification of E. coli proteins binding to pAp: CysQ and Orn.

### 1-CysQ is a main target for lithium toxicity in E. coli

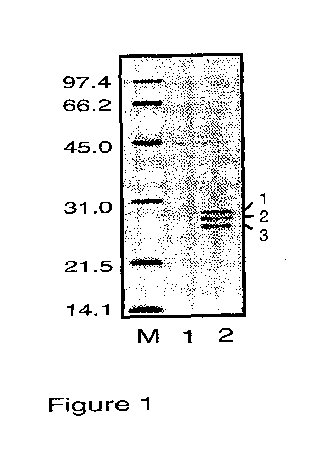

As an approach to explore the regulatory function of pAp, the inventors chose the identification of proteins interacting with this nucleotide. Initial binding experiments using pAp-agarose as affinity matrix were done on extracts of *E. coli* CC118. This strain harbors a deletion for alkaline phosphatase in order to prevent degradation of the affinity substrate. Three major protein bands became visible in the fraction of proteins binding specifically to pAp (Figure 1). Analysis of bands number 1 and 3 by LC-MS/MS revealed CysQ and oligoribonuclease, Orn, respectively.

CysQ was identified with an overall score of 860 represented by 5 distinct peptides, covering 22.3% of the total mass of the protein. Orn was identified with an overall score of 768, eight peptides covering 42% of the total protein mass were identified.

The inventors demonstrate that CysQ is a main target for lithium toxicity in *E. coli.* Like its homologue in yeast (Met22/Hal2p) (Murguia et al, 1995) and the mammalian counterparts (BPNT1) (Lopez-Coronado et al, 1999; Spiegelberg et al, 1999), CysQ was shown to degrade pAp to AMP (Spiegelberg et al, 1999). Recovery of CysQ validates the approach of the inventors, as it shows that cross linking to the matrix does not interfere with major features of pAp as a substrate for specific interactions.

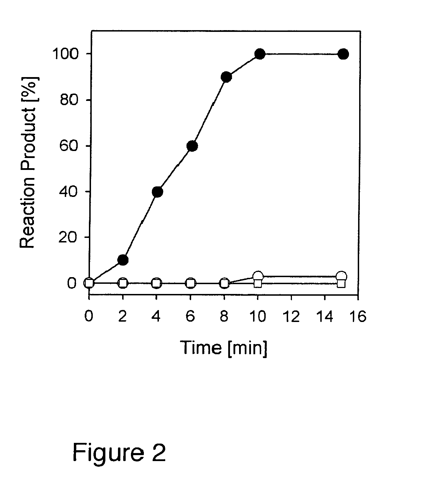

CysQ carries a conserved sequence motif, D(X)ₙEE(X)ₙDP(I/L)D(S/G/A)T(X)ₙWD(X)₁₁GG required for metal ion coordination that places it into a larger family of magnesium-dependent phosphomonoesterases with otherwise low overall sequence similarity but a conserved three dimensional core structure (York *et al.,* 1995). Members of this family are predicted to be highly sensitive to lithium, but this requires experimental substantiation. To test lithium sensitivity of CysQ, the his-tagged protein was purified to homogeneity. As expected, CysQ-catalyzed pAp hydrolysis requires Mg²⁺. Sensitivity to calcium was reported for pAp phosphatases. For this reason it has been included CaCl₂ in the test. Figure 2 shows complete inhibition of CysQ in the presence of either 1 mM LiCl or 2 mM CaCl₂. Reactions were performed in the presence of 200 mM NaCl. Under these conditions CysQ activity was higher than in the absence of NaCl. Thus, sodium is not inhibitory at physiological concentrations and does not interfere with lithium. Importantly, this places CysQ in the class of sodium tolerant pAp phosphatases, together with mammalian pAp phosphatases rat BPNT1 (Lopez-Coronado et al, 1999) and human BPNT1 (Yenush et al, 2000) and two homologous proteins in Arabidopsis (At-Sal1 and At-Sal2) (Gil-Mascarell et al, 1999), and contrasts it with the yeast proteins Met22/Ha12P (Murguia et al, 1996) and Tol1 (Miyamoto et al, 2000) and the third pAp-phosphatase in Arabidopsis, At-AHL (Gil-Mascarell et al, 1999).

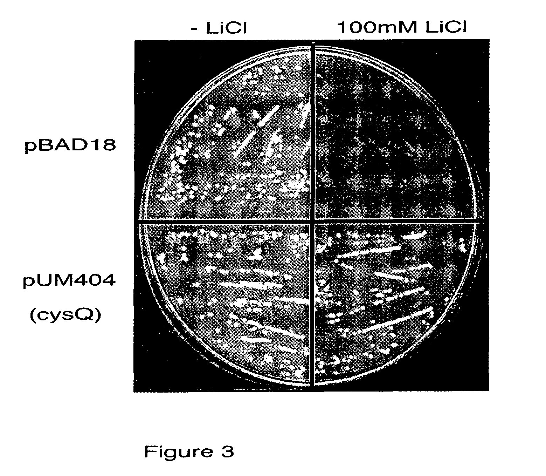

Despite the enormous phylogenetic distance between *E. coli* and humans, sensitivity to low concentrations of LiCl makes CysQ a potential target for lithium-toxicity in *E. coli* in vivo. To test this hypothesis, the wild-type strain MG1655 was grown in the presence of sulfate as sole sulfur source. Under these conditions, cysteine needs to be synthesized through the assimilation of sulfate, and pAp is produced as a by-product. Adding LiC1 should inhibit pAp phosphatase and lead to an accumulation of pAp. It was observed a considerable inhibitory effect of lithium on cell growth. Differences in colony size were observed already after plating cells in the presence of 50 mM LiC1. Severe growth inhibition was observed at 100 mM LiC1 (Figure 3, top). Importantly, this growth defect could be almost completely rescued by overexpression of CysQ (Figure 3, bottom). The present invention therefore shows that CysQ is a main target for Li-toxicity under the conditions used.

### 2- Orn is a protein specifically binding to pAp.

As lithium-toxicity is caused mainly by accumulation of pAp, the question arises what are the downstream targets of pAp and/or pAp-accumulation. It is expected that targets should be found among the proteins binding to pAp. The inventors identified Orn as one of these proteins.

Orn is an essential protein in *E. coli* involved in mRNA degradation. It has 3' to 5' exoribonuclease activity selectively active on short ribonucleotides. 5'adenosine monophosphate (AMP) is known to inhibit Orn (Datta & Niyogi, 1975). It was therefore important to compare binding of Orn to AMP and pAp. To this end recombinant Orn has been purified and its binding was measured to equal amounts of pAp- or AMP-agarose, both affinity substrates with identical binding capacities, CysQ was included as a positive control. Similar experiments were done with cell free extracts from strains overexpressing Orn or CysQ. As seen in Figure 4, both recombinant Orn and CysQ interact strongly with pAp, 15 µg of protein binding almost quantitatively to 5 µl of pAp-agarose. Furthermore, significant amounts of Orn, roughly one third, but not CysQ bind to AMP-agarose. This preferential binding of Orn to pAp over AMP held true in the presence of competing proteins when cellular extracts of cultures overexpressing Orn were used for binding experiments. Moreover, the use of pAp affinity chromatography appears to be an effective purification step of both Orn or CysQ from extracts of cells overexpressing these proteins.

### 3- Orn is strongly inhibited by pAp in vitro.

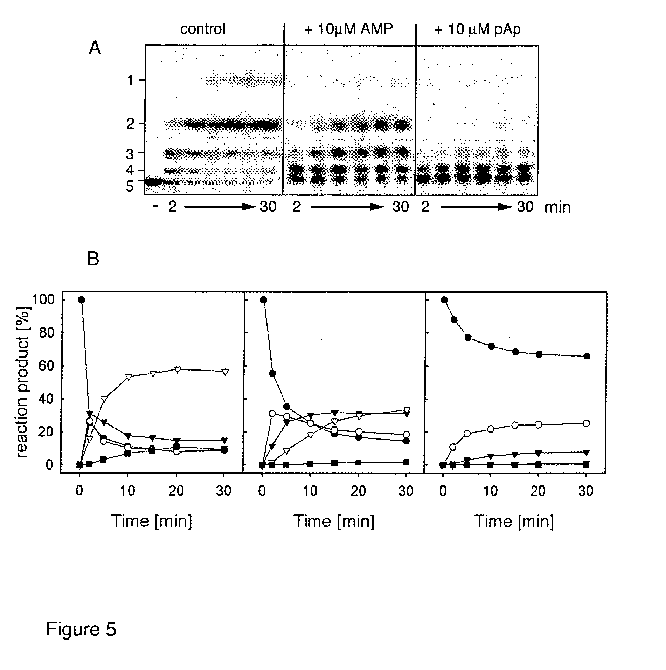

Given the strong affinity of Orn for pAp, it was investigated whether pAp affects Orn activity. Orn was shown to be most active on single-stranded RNA oligonucleotides of ≤5 nucleotides in length. Those small RNA-oligos are called here nanoRNAs, in order to distinguish them from microRNAs, which have become a subject of extensive studies in the last several years. RNA-oligo 5-mers (5'-CCCCC-3') labeled at their 5'-phosphate end with the sulfoindocyanine succinimidyl ester cyanine 5 (Cy5) were therefore used as substrate for in vitro assays with purified recombinant Orn. Separating reaction products on a 22% SDS-Polyacrylamide (PAA) gel, it was observed a reverse migration phenomenon (Figure 5). This effect can be accounted for by the fact that cyanine dyes have a lower net negative charge than nucleic acids: thus, removing nucleotides will reduce the charge relative to the mass of the oligonucleotide and cause it to shift up instead of down. As could be expected from previous reports (Datta & Niyogi, 1975), AMP was inhibiting the degradation of nanoRNA at relatively low concentrations (10 µM, Figure 5, middle). However, the effect of 10 µM pAp was significantly more pronounced (Figure 5, right). The inventors concluded that pAp is a more potent inhibitor of Orn than its known inhibitor AMP. The sequential appearance of reaction products of different chain length (Figure 5, B) is in agreement with previous data (Datta & Niyogi, 1975) and the proposed processive reaction mechanism of oligoribonuclease. The preferential appearance of oligo-4mers in the presence of pAp points to possible effects of this nucleotide on enzyme processivity.

### EXAMPLE 2: Identification of pAp- binding polypeptides in human cells

Cytoplasmic extracts of HeLa cells were dialyzed against pAp-binding buffer and concentrated (10-fold) before binding experiments were performed in a similar way as described for bacterial extracts. Figure 6 shows the presence of three major proteins that bind specifically to pAp. Identification of bands number 2 and number 3 by LC/MS-MS revealed nucleoside diphosphate kinase (Ndk) with an overall score of 158, and 4 peptides covering 16% of the total mass for the lowest band (number 1) and an overall score of 280, 6 peptides covering 35% of the total mass for band number 2. The peptides identified for band number 1 were, TFIAIKPDGVQR, PDGVQR, which is contained in peptide 1 and the result of a more complete trypsin digestion, GDFCIQVGR, and GLVEIIK. Band number 2 was identified by the following peptides: DRPFFAGLVK, FMQASEDLLK, GDFCIQVGR, GLVEIIK, NIIHCSDSVESAEK, and TFIAIKPDGVQR. Ndk exists in different isoforms and different functions have been reported for individual isoforms. The peptides identified for bands number 1 and number 2 do not allow distinguishing unequivocally between these isoforms, differences in the molecular weight however point to isoform b for band number 1 with a predicted molecular weight of 17.1 kD and isoform a for band number 2 (19.6 kD).

Ndk was reported to be inhibited by 3'-phosphorylated nucleotides, in particular by pAp and PAPS (Schneider et al, 1998).

A downstream target of pAp accumulation is conserved between *E. coli* and humans: Sfn.

Identification of band number 3 revealed small fragment nuclease (Sfn) with an overall score of 338, and 7 peptides covering 31 % of the total mass. Sfn is the human homologue of *E. coli* Orn and shares a high degree of similarity (50%) with its bacterial counterpart (Koonin, 1997). Sfn and Orn belong to a large family of 3'-5' exonucleases that are characterized by three ancient conserved exonuclease motifs (exo I, II, and III) (Bernad et al, 1989; Koonin & Deutscher, 1993). As Orn, Sfn specifically degrades short oligoribonucleotides of ≤5 nucleotides in length. In addition, Sfn was shown to be active on small DNA-oligos (Nguyen et al, 2000). The inventors have demonstrated the role of Sfn in cellular nucleotide recycling. The inventors' recognition of Sfn as pAp-binding polypeptide demonstrates that, besides pAp phosphatase, at least one target of pAp and/or pAp-accumulation is conserved between *E. coli* and humans.

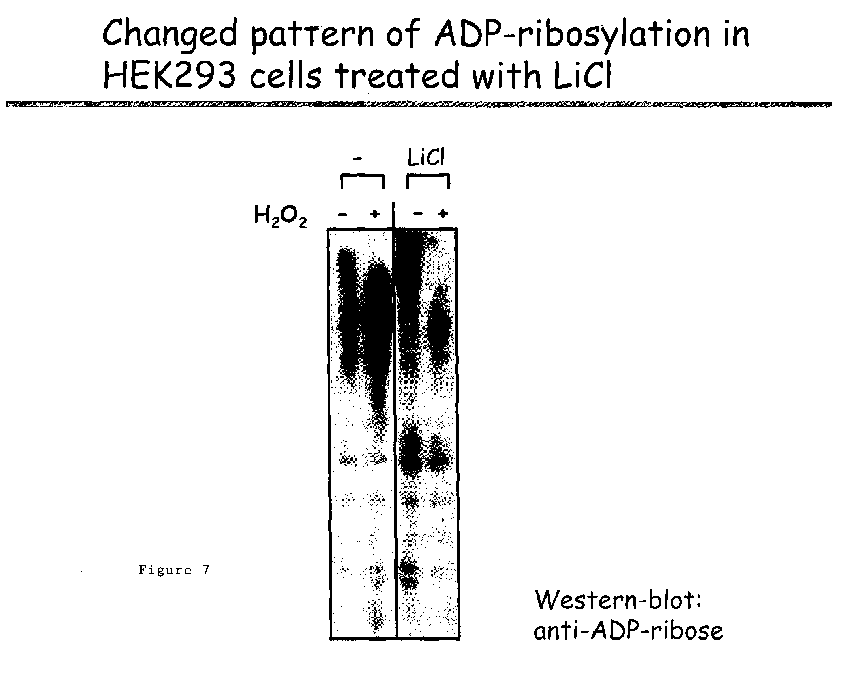

Figure 7, showing the human proteins binding to pAp-agarose, indicates in the nuclear extract the presence of a polypeptide named PARP.

### References

Bachmann B. (1996) In Escherichia coli and Salmonella: Cellular and Molecular Biology. ASM Press, pp. 2460-2488.
Bernad A, Blanco L, Lazaro JM, Martin G, Salas M. (1989) A conserved 3'-5' exonuclease active site in prokaryotic and eukaryotic DNA polymerases. Cell 59: 219-228.
Berridge MJ, Downes CP, Hanley MR. (1989) Neural and developmental actions of lithium: a unifying hypothesis. Cell 59: 411-419.
Cade J. (1949) Lithium salts in the treatment of psychotic excitement. Med J Aust 36: 349-352.
Cimarosti et al. (2001) An investigation of the neuroprotective effect of lithium in organotypic slice cultures of rat hippocampus exposed to oxygen and glucose deprivation, Neuroscience Letters 315: 33-38
Datta AK, Niyogi K. (1975) A novel oligoribonuclease of Escherichia coli. II. Mechanism of action. J Biol Chem 250: 7313-7319.
Dignam JD, Martin PL, Shastry BS, Roeder RG. (1983) Eukaryotic gene transcription with purified components. Methods Enzymol 101: 582-598.
Falany CN. (1991) Molecular enzymology of human liver cytosolic sulfotransferases. Trends Pharmacol Sci 12: 255-259.
Ghosh S, Deutscher MP. (1999) Oligoribonuclease is an essential component of the mRNA decay pathway. Proc Natl Acad Sci U S A 96: 4372-4377.
Gil-Mascarell R, Lopez-Coronado JM, Belles JM, Serrano R, Rodriguez PL. (1999) The Arabidopsis HAL2-like gene family includes a novel sodium-sensitive phosphatase. Plant J 17: 373-383.
Glaser HU, Thomas D, Gaxiola R, Montrichard F, Surdin-Kerjan Y, Serrano R. (1993) Salt tolerance and methionine biosynthesis in Saccharomyces cerevisiae involve a putative phosphatase gene. Embo J 12: 3105-3110.
Guzman LM, Belin D, Carson MJ, Beckwith J. (1995) Tight regulation, modulation, and high-level expression by vectors containing the arabinose PBAD promoter. J Bacteriol 177: 4121-4130.
Hallcher LM, Sherman WR. (1980) The effects of lithium ion and other agents on the activity of myo-inositol-1-phosphatase from bovine brain. J Biol Chem 255: 10896-10901.
Hartsough MT, Steeg PS. (2000) Nm23/nucleoside diphosphate kinase in human cancers. J Bioenerg Biomembr 32: 301-308.
Ito S, Kita K, Zhai L, Wano C, Suzuki T, Yamaura A, Suzuki N. (2004) Involvement of human small fragment nuclease in the resistance of human cells to UV-C-induced cell death. Photochem Photobiol 80: 281-285.
Jagtap et al. (2005), Poly(ADP-ribose) polymerase and the therapeutic effects of its inhibitors, Nature Reviews Drug discovery 4, 421-440.
Klaassen CD, Boles JW. (1997) Sulfation and sulfotransferases 5: the importance of 3'-phosphoadenosine 5'-phosphosulfate (PAPS) in the regulation of sulfation. Faseb J 11: 404-418.
Klein PS, Melton DA. (1996) A molecular mechanism for the effect of lithium on development. Proc Natl Acad Sci U S A 93: 8455-8459.
Koonin EV. (1997) A conserved ancient domain joins the growing superfamily of 3'-5' exonucleases. Curr Biol 7: R604-606.
Koonin EV, Deutscher MP. (1993) RNase T shares conserved sequence motifs with DNA proofreading exonucleases. Nucleic Acids Res 21: 2521-2522.
Lacombe ML, Milon L, Munier A, Mehus JG, Lambeth DO. (2000) The human Nm23/nucleoside diphosphate kinases. J Bioenerg Biomembr 32: 247-258.
Lenox RH, Wang L. (2003) Molecular basis of lithium action: integration of lithium-responsive signaling and gene expression networks. Mol Psychiatry 8: 135-144.
Lombardi D, Lacombe ML, Paggi MG. (2000) nm23: unraveling its biological function in cell differentiation. J Cell Physiol 182: 144-149.
Lopez-Coronado JM, Belles JM, Lesage F, Serrano R, Rodriguez PL. (1999) A novel mammalian lithium-sensitive enzyme with a dual enzymatic activity, 3'-phosphoadenosine 5'-phosphate phosphatase and inositol-polyphosphate 1-phosphatase. J Biol Chem 274: 16034-16039.
Manoil C, Beckwith J. (1985) TnphoA: a transposon probe for protein export signals. Proc Natl Acad Sci U S A 82: 8129-8133.
Miyamoto R, Sugiura R, Kamitani S, Yada T, Lu Y, Sio SO, Asakura M, Matsuhisa A, Shuntoh H, Kuno T. (2000) Toll, a fission yeast phosphomonoesterase, is an in vivo target of lithium, and its deletion leads to sulfite auxotrophy. J Bacteriol 182: 3619-3625.
Murguia JR, Belles JM, Serrano R. (1995) A salt-sensitive 3'(2'),5'-bisphosphate nucleotidase involved in sulfate activation. Science 267: 232-234.
Murguia JR, Belles JM, Serrano R. (1996) The yeast HAL2 nucleotidase is an in vivo target of salt toxicity. J Biol Chem 271: 29029-29033.
Nguyen LH, Erzberger JP, Root J, Wilson DM, 3rd. (2000) The human homolog of Escherichia coli Orn degrades small single-stranded RNA and DNA oligomers. J Biol Chem 275: 25900-25906.
Niyogi SK, Datta AK. (1975) A novel oligoribonuclease of Escherichia coli. I. Isolation and properties. J Biol Chem 250: 7307-7312.
Postel EH. (1998) NM23-NDP kinase. Int J Biochem Cell Biol 30: 1291-1295.
Postel EH. (1999) Cleavage of DNA by human NM23-H2/nucleoside diphosphate kinase involves formation of a covalent protein-DNA complex. J Biol Chem 274: 22821-22829.
Postel EH, Abramczyk BA, Gursky SK, Xu Y. (2002) Structure-based mutational and functional analysis identify human NM23-H2 as a multifunctional enzyme. Biochemistry 41: 6330-6337.
Postel EH, Abramczyk BM. (2003) Escherichia coli nucleoside diphosphate kinase is a uracil-processing DNA repair nuclease. Proc Natl Acad Sci U S A 100: 13247-13252.
Postel EH, Abramczyk BM, Levit MN, Kyin S. (2000a) Catalysis of DNA cleavage and nucleoside triphosphate synthesis by NM23-H2/NDP kinase share an active site that implies a DNA repair function. Proc Natl Acad Sci U S A 97: 14194-14199.
Postel EH, Berberich SJ, Flint SJ, Ferrone CA. (1993) Human c-myc transcription factor PuF identified as nm23-H2 nucleoside diphosphate kinase, a candidate suppressor of tumor metastasis. Science 261: 478-480.
Quintero FJ, Garciadeblas B, Rodriguez-Navarro A. (1996) The SAL1 gene of Arabidopsis, encoding an enzyme with 3'(2'),5'-bisphosphate nucleotidase and inositol polyphosphate 1-phosphatase activities, increases salt tolerance in yeast. Plant Cell 8: 529-537.
Quiroz JA, Gould TD, Manji HK. (2004) MOLECULAR EFFECTS of lithium. Mol Interv 4: 259-272.
Reuter K, Mofid MR, Marahiel MA, Ficner R. (1999) Crystal structure of the surfactin synthetase-activating enzyme sfp: a prototype of the 4'-phosphopantetheinyl transferase superfamily. Embo J 18: 6823-6831.
Schneider B, Xu YW, Janin J, Veron M, Deville-Bonne D. (1998) 3'-Phosphorylated nucleotides are tight binding inhibitors of nucleoside diphosphate kinase activity. J Biol Chem 273: 28773-28778.
Shaldubina A, Agam G, Belmaker RH. (2001) The mechanism of lithium action: state of the art, ten years later. Prog Neuropsychopharmacol Biol Psychiatry 25: 855-866.
Skaper SD. (2003) Poly(ADP-ribosyl)ation enzyme-1 as a target for neuroprotection in acute central nervous system injury, Curr Drug Targets CNS Neurol Disord., October; 2(5):279-91.
Skaper SD. (2003) Poly(ADP-Ribose) polymerase-1 in acute neuronal death and inflammation: a strategy for neuroprotection. Ann NY Acad Sci. 2003 May;993:217-28.
Spiegelberg et al. (1999) Cloning and Characterization of a Mammalian Lithium-sensitive Bisphosphate 3'-Nucleotidase Inhibited by Inositol 1,4-Biosphosphate, The Journal of Biological Chemistry: vol.274, n° 10, 13619-13628.
Stambolic V, Ruel L, Woodgett JR. (1996) Lithium inhibits glycogen synthase kinase-3 activity and mimics wingless signalling in intact cells. Curr Biol 6: 1664-1668.
Venturelli D, Martinez R, Melotti P, Casella I, Peschle C, Cucco C, Spampinato G, Darzynkiewicz Z, Calabretta B. (1995) Overexpression of DR-nm23, a protein encoded by a member of the nm23 gene family, inhibits granulocyte differentiation and induces apoptosis in 32Dc13 myeloid cells. Proc Natl Acad Sci U S A 92: 7435-7439.
Weinshilboum R, Aksoy I. (1994) Sulfation pharmacogenetics in humans. Chem Biol Interact 92: 233-246.
Weinshilboum RM. (1986) Sulfate conjugation of neurotransmitters and drugs. Introduction. Fed Proc 45: 2220-2222.
Yenush L, Belles JM, Lopez-Coronado JM, Gil-Mascarell R, Serrano R, Rodriguez PL. (2000) A novel target of lithium therapy. FEBS Lett 467: 321-325.
York JD, Ponder JW, Majerus PW. (1995) Definition of a metal-dependent/Li(+)-inhibited phosphomonoesterase protein family based upon a conserved three-dimensional core structure. Proc Natl Acad Sci U S A 92: 5149-5153.
Yu D, Deutscher MP. (1995) Oligoribonuclease is distinct from the other known exoribonucleases of Escherichia coli. J Bacteriol 177: 4137-4139.

## Claims

1. A complex formed between the 3'-phosphoadenosine 5'-phosphate (pAp) and a polypeptide in human cells.

2. Complex according to claim 1 wherein the polypeptide is the Poly (ADP-ribose) Polymerase (PARP).

3. Complex according to claim 1 wherein the polypeptide is the Small fragment nuclease (Sfn).

4. Complex according to anyone of claim 1 to 3 which is a purified complex.

5. Use of a molecule which modulates the formation of the pAp-PARP complex according to claim 2 or pAp-Sfn complex according to claim 3, for the manufacture of a medicament for preventing or treating bipolar disorders and/or side effects linked to lithium administration.

6. A process for screening a molecule which modulates or mimics at least one effect of lithium in human, said process comprising:
a) incubating a polypeptide which complexes with pAp in human cells with said molecule,
b) measuring the activity of said polypeptide,
wherein a modulating or mimic of the activity of said polypeptide indicates that said molecule modulates or mimics at least one effect of lithium in human.

7. The process according to claim 6 for screening a molecule which mimics at least one effect of lithium in human, said process comprising:
a) incubating a polypeptide which complexes with pAp in human cells with said molecule,
b) measuring the activity of said polypeptide,
wherein a modulating of the activity of said polypeptide which is comparable with the modulating of the activity of said polypeptide in the presence of pAp in the cell indicates that said molecule mimics at least one effect of lithium in human.

8. The process according to claim 6 for screening a molecule which modulates at least one effect of lithium in human, said process comprising:
a) incubating a polypeptide which complexes with pAp in human cells with said molecule in the presence of pAp in the cell,
b) measuring the activity of said polypeptide,
wherein a modulating of the activity of said polypeptide compared with the activity of said polypeptide in the presence of pAp in a cell indicates that said molecule modulates at least one effect of lithium in human.

9. The process according to claim 7 or 8, wherein the presence of Pap in the cell derived from the presence of lithium.

10. The process according to claim 6, wherein said polypeptide is PARP.

11. The process according to claim 10, wherein said effect of lithium in human is degradation of RNA-oligos and/or DNA-oligos.

12. The process according to claim 6, wherein said polypeptide is Sfn.

13. The process according to claim 12, wherein said effect of lithium in human is modulation of DNA repair.

14. Process according to anyone of claims 6 to 13, wherein the molecule is a candidate drug for the treatment of bipolar disorder.

15. Use of a molecule screened in the process according to anyone of claims 6 to 13 as a medicament.

16. Use of a molecule screened in the process according to anyone of claims 6 to 13 for the manufacture of a medicament for treatment of bipolar disorder.

17. Use of pAp or of a molecule which mimics pAp for modulating DNA repair and/or for degrading RNA-oligos and/or DNA-oligos.

18. Use according to claim 17, for the manufacture of a medicament for preventing or treating diseases where a modulation of PARP activity is needed, such as cancer, cell necrosis, inflammation, neurodegenerative diseases or tissue injury.

19. Use according to claim 18, wherein PARP activity is inhibited.

20. Use according to claim 17, for modulating of Sfn activity.

21. Use according to claim 17, for the manufacture of a medicament for preventing or treating bipolar disorders and/or side effects linked to lithium administration.

22. Downstream target polypeptide of pAp in human cells for use as a medicament.

23. Use of the downstream target polypeptide of pAp according to claim 22 for the manufacture of a medicament for treating bipolar disorder.

24. Use of the downstream target polypeptide of pAp according to claim 23, wherein it is selected from Sfn and PARP.


SEQUENCE LISTING

<110> INSTITUT PASTEUR
      CENTRE NATIONAL DE LA RECHERCHE SCIENTIFIQUE (CNRS)

<120> Process of screening a test substance as a candidate drug
      for the treatment of bipolar disorder.

<130> 66144 / D23675

<160> 7

<170> PatentIn version 3.1

<210> 1
<211> 12
<212> PRT
<213> Homo sapiens

<220>
<223> Peptide identified for band number 1 and 2 (figure 6)

<400> 1

Thr Phe Ile Ala Ile Lys Pro Asp Gly Val Gln Arg
1               5                   10


<210> 2
<211> 6
<212> PRT
<213> Homo sapiens

<220>
<223> Peptide identified for band number 1 (figure 6)

<400> 2

Pro Asp Gly Val Gln Arg
1               5


<210> 3
<211> 9
<212> PRT
<213> Homo sapiens

<220>
<223> Peptide identified for band number 1 and 2(figure 6)

<400> 3

Gly Asp Phe Cys Ile Gln Val Gly Arg
1               5


<210> 4
<211> 7
<212> PRT
<213> Homo sapiens

<220>
<223> Peptide identified for band number 1 and 2 (figure 6)

<400> 4

Gly Leu Val Glu Ile Ile Lys
1               5


<210> 5
<211> 10
<212> PRT
<213> Homo sapiens

<220>
<223> Peptide identified for band number 2 (figure 6)

<400> 5

Asp Arg Pro Phe Phe Ala Gly Leu Val Lys
1               5                   10


<210> 6
<211> 10
<212> PRT
<213> Homo sapiens

<220>
<223> Peptide identified for band number 2 (figure 6)

<400> 6

Phe Met Gln Ala Ser Glu Asp Leu Leu Lys
1               5                   10


<210> 7
<211> 14
<212> PRT
<213> Homo sapiens

<220>
<223> Peptide identified for band number 2 (figure 6)

<400> 7

Asn Ile Ile His Cys Ser Asp Ser Val Glu Ser Ala Glu Lys
1               5                   10